# EUROPEAN PATENT SPECIFICATION

(11) **EP 4 460 265 B1**
(45) Date of publication and mention of the grant of the patent: **11.11.2026**
(21) Application number: 23713302.0
(22) Date of filing: 09.01.2023
(51) Int. Cl.: A61F 2/46

(54) **IMPROVEMENTS IN AND RELATING TO DEVICES FOR SURGICAL INSTRUMENT IMPACTION**
VERBESSERUNGEN AN UND IM ZUSAMMENHANG MIT VORRICHTUNGEN ZUR AUSWIRKUNG AUF CHIRURGISCHE INSTRUMENTE
AMÉLIORATIONS APPORTÉES ET RELATIVES À DES DISPOSITIFS D'IMPACTION POUR INSTRUMENT CHIRURGICAL

(30) Priority: 07.01.2022 GB 202200195
(43) Date of publication of application: 13.11.2024
(73) Proprietor: DePuy Ireland Unlimited Company, Ringaskiddy, County Cork (IE)
(72) Inventor: ATKIN, Jamie, Leeds Yorkshire LS11 8DT (GB); DUTTON, Graeme, Leeds Yorkshire LS11 8DT (GB)
(74) Representative: Murgitroyd & Company
(86) International application number: PCT/EP2023/050292
(87) International publication number: WO 2023/131700

(56) References cited:
- US-A- 5 514 136
- US-A1- 2006 095 043
- US-A1- 2009 099 566
- US-A1- 2021 353 430

## Description

### TECHNICAL FIELD

The present disclosure relates generally to orthopaedic devices, kits including such devices and methods of using such devices and more particularly to orthopaedic surgical instrument impaction devices, kits including such devices and methods of using such devices.

### BACKGROUND

Joint arthroplasty is a well-known surgical procedure by which a diseased and/or damaged natural joint is replaced by a prosthetic joint. Examples include knee prosthesis and hip implants. Surgical instruments are used to prepare the bone for the receipt of further surgical instruments and/or implants and a reliable impaction device for those surgical instruments is desirable.

US 2009/099566 A1 discloses a modular stem inserter for use in arthroplasty including a handle and various detachable and interchangeable shafts. US 5514136 discloses an instrument for driving and rotating a long bone prosthesis. US 2006/095043 A1 discloses a surgical instrument inserter device with interchangeable heads to allow use in different positions and/or configurations. US 2021/353430 A1 discloses devices for the extraction of a prosthetic component during a surgical procedure.

### SUMMARY

According to a first aspect of the invention there is provided a surgical instrument inserter device, the inserter device comprising:
a body;
a first mounting location provided at the proximal end of the body;
a second mounting location provided at the distal end of the body;
wherein the second mounting location includes a contact element for the surgical instrument to be inserted in use, the contact element having one or more non-planar contact surface sections; wherein one or more or all of the contact surface sections are provided by a deformed partial sphere having reduced radii in a first set of directions compared with radii in a second set of directions.

The contact element may include a plurality of separate non-planar contact surface sections. Particularly, the contact element may provide a single non-planar contact surface. One or more or all of the non-planar contact surfaces may be articulation surfaces. One or more or all of the non-planar contact surface sections may be curved contact surface sections and/or part-spherical contact surface sections. One or more or all of the contact surface sections may be provided by a continuous surface. The deformed partial sphere may be partial due to the absence of a part of the sphere, for instance due to a section to one side of a surface, such as a planar surface, being removed. The deformed partial sphere may be a compressed sphere, for instance with the compression along one axis.

One or more non-planar contact surface sections may be provided adjacent the junction between the second mounting location and the body, for instance adjacent the junction between the contact element and the elongate portion.

One or more non-planar contact surface sections may be provided distal the junction between the second mounting location and the body, for instance opposite the junction between the contact element and the elongate portion. One or more non-planar contact surface sections may be provided opposite the junction between the second mounting location and the body, for instance opposite the junction between the contact element and the elongate portion.

The contact element may comprise one or more other contact surface sections. The contact element may comprise one or more near planar or planar other contact surface sections. The one or more other contact surface sections may be sections of the same continuous surface as the one or more non-planar contact surface sections are part of.

The one or more other contact surface sections may be provided in one or both polar regions, for instance along an axis through the contact element, particularly an axis perpendicular to the axis of the longitudinal axis of the inserter device. The contact element may comprise two flattened other contact surface sections, for instance flattened compared with the curvature of the one or more non-planar contact surface sections. The two flattened other contact surface sections may be in opposition to one another. The contact element may comprise two planar other surface sections. The contact element may comprise at least two planar other surfaces which are coplanar with one another and/or extend parallel to the longitudinal axis of the inserter device.

The contact element may have the profile of a sphere with one or more spherical caps removed. The plane defining a first spherical cap removed may be the plane forming the junction between the contact element and the elongate portion. The plane defining a second spherical cap removed may be a first planar other surface, for instance which is coplanar with a second planar other surface and/or extends parallel to the longitudinal axis of the inserter device. The plane defining a third spherical cap removed may be a second planar other surface, for instance which is coplanar with a first planar other surface and/or extends parallel to the longitudinal axis of the inserter device. The plane defining the first spherical cap removed may touch and/or intersect the plane defining the second spherical cap removed. The plane defining the first spherical cap removed may touch and/or intersect the plane defining the third spherical cap removed.

The contact element may have a first extent between a first pair of points on opposite sides of the contact element and have a second extent between a second different pair of points on different opposite sides of the contact element, wherein the first extent is greater than the second extent.

The contact element may have a first extent, for instance between a first pair of points on opposite sides of the contact element. The contact element may have a second extent, for instance between a second different pair of points on opposite sides of the contact element.

The first extent may be greater than the second extent. The first extent may be at least 5% greater than the second extent. The first extent may be at least 10%, or at least 15% or at least 20% greater than the second extent.

The first extent may be between a first pair of points on two different non-planar surfaces, for instance on two non-planar surfaces which are in opposition to one another and/or on two non-planar surfaces which are on opposite sides of the contact element to one another.

The second extent may be between a second pair of points on two different near planar or planar surface sections, for instance on two near planar or planar surfaces which are coplanar with one another and/or extend parallel to the longitudinal axis of the inserter device.

The first extent and/or the second extent may be considered along an axis and/or in a plane, perpendicular to the longitudinal axis of the inserter device and/or of the body portion. The first extent may be considered along an axis perpendicular to a point on a first non-planar surface and/or perpendicular to a point on a second non-planar surface. The second extent may be considered along an axis and/or in a plane, perpendicular to the plane of a first planar surface section and/or perpendicular to the plane of a second surface section.

References in this document to perpendicular to, may in one or more or all of those instances include perpendicular to +/- 10°, or +/- 8°, or +/- 5°, or +/- 3°.

The body may include a body portion. The body may include an elongate portion. The body may include a transition portion. The transition portion may connect the body portion to the elongate portion.

The first mounting location may be provided by the body portion, particularly on the proximal end of the body portion.

The second mounting location may be provided on the elongate portion, particularly on the distal end of the elongate portion.

The first mounting location may be adapted to connect the inserter device to the distal end of a tool.

The first mounting location may include a stem. The stem may extend from the body, for instance from the body portion, along an axis which is coaxial to the body portion and/or proximal part of the elongate portion. The first mounting location may provide a recess to receive one or more parts of the tool. The first mounting location may provide a recess defined in part by the proximal end of the stem. The stem may be provided with one or more alignment surfaces. The stem may be provided with one or more non-circular, for instance planar, alignment surfaces. The alignment surfaces may extend parallel to but spaced from the axis of the stem.

The first mounting location may be adapted to connect, for instance insert into, a distal end of a tool. The first mounting location may be adapted to engage with a latch type mechanism on the tool, for instance a releasable protrusion into the recess in the stem.

The first mounting location may have a tool engaged state and a tool disengaged state. The first mounting location may transition from the tool engaged state to the tool disengaged state and/or from the tool disengaged state to the tool engaged state by relative axial movement of the tool and the inserter device.

The inserter device may have a longitudinal axis, for instance adapted to align with a longitudinal axis of the tool.

The body may include a first cross-sectional area part and a second cross-sectional area part, which is closer to the distal end of the inserter device. The body portion may include the first cross-sectional area part. The elongate portion may include the second cross-sectional area part. The body may include a third cross-sectional area part. The transition part may include the third cross-sectional area part.

The first cross-sectional area part may have a greater cross-sectional area than the second cross-sectional area part. The first cross-sectional area part may have a greater cross-sectional area than the third cross-sectional area part.

The first cross-sectional area part may have a circular cross-sectional area. The first cross-sectional area part may have a partially circular cross-sectional area. The first cross-sectional area part may include two or more linear parts to its perimeter. Two or more linear parts may be provided in opposition to one another and/or parallel to one another.

The second cross-sectional area part may lead to the second mounting location, particularly at the distal end thereof.

The second cross-sectional area part may have a lesser cross-sectional area than the third cross-sectional area part.

The second cross-sectional area part may have a circular cross-sectional area, at least along a part of its length, for instance along a linear section.

The third cross-sectional area part may have a circular cross-sectional area.

The body, for instance the body portion, may be or include a cylindrical portion. The body portion may include one or more planar surfaces. One or more pairs of planar surfaces may be provided, for instance with the planar surfaces of the pair in opposition to one another. The planar surfaces may be provided parallel to the longitudinal axis of the inserter device.

The body, for instance the transition portion, may be or include a conical portion. The transition portion may transition from the shape and configuration of the body portion to the shape and configuration of the elongate portion.

The body, for instance the elongate portion, may be or include a cylindrical portion. The body, for instance the elongate portion, may be or include a neck portion.

The elongate portion may have a linear section provided between the proximal end and the distal end. The elongate portion may extend from at least adjacent to the proximal end. The linear section may extend from the proximal end.

The elongate portion may have a linear section and a non-linear section provided between the proximal end and the distal end. The non-linear section may extend from the linear section towards the distal end. The non-linear section may extend to the distal end or adjacent to the distal end. The non-linear section may extend to the junction between the elongate portion and the second mounting location.

The non-linear section may include a first linear part, a curved part and a second linear part. The non-linear section may include a curved part. The curved part may bend away from a first axis in a first part. The curved part may bend towards a second axis in a second part. The second axis may be parallel to, but spaced from, the first axis. The first axis may be coaxial with the longitudinal axis of the first linear part and/or the elongate portion and/or inserter device and/or tool. The second axis may be offset relative to with the longitudinal axis of the first linear part and/or the elongate portion and/or inserter device and/or tool. The axis of compression of the contact element may lie on and be perpendicular to the second axis. The curved part may have an intermediate part to the first part and the second part. The intermediate part may be curved. The intermediate part may include an inflection location between the curve away and the curve towards. The intermediate part may be or may include a linear part, for instance to contribute to the offset of the second axis from the first axis.

The non-linear section may include a first linear part, an intermediate linear part and a second linear part. The non-linear section may include an intermediate linear part, the intermediate linear part may provide the non-linear section by being inclined relative to the longitudinal axis of the first linear part and/or of the elongate portion and/or of the inserter device and/or of the tool.

In an alternative embodiment, the elongate portion may have a linear section, with the linear section extending from a proximal end to a distal end, potentially with the second mounting location provided by or connected to the distal end. The proximal end and/or distal end and/or second mounting location may lie on a common axis, for instance an axis common with the longitudinal axis of the tool in use.

The elongate part may have a first cross-section, for instance a circular cross-section. The first cross-section may be provided along the length of the elongate part for at least 60% of the distance from the proximal end of the elongate part towards the distal end of the elongate part, particularly starting from the proximal end.

The elongate part may have a second cross- section, for instance a non-circular cross-section. The second cross-section may be provided along the length of the elongate part for at least 10% of the distance from the distal end of the elongate part towards the proximal end of the elongate part, particularly starting from the distal end. The second cross-section may include one or more flats providing part of the perimeter of the cross-section, for instance two flats in opposition to one another.

The elongate part, particularly the distal end section thereof, may be provided with a planar surface, such as a flat, extending there along. The elongate part, particularly the distal end section thereof, may be provided with a pair of opposing planar surfaces.

The distal end of the elongate part, for instance at the junction between the elongate portion and the second mounting location, may be provided with a different cross-section to the elongate section. The distal end of the elongate part, for instance at the junction between the elongate portion and the second mounting location, may be provided with a different cross-section to the first cross-section and/or to the second cross-section. The different cross-section may be an oval, for instance with linear sides. The different cross-section may provide a flared cross-section compared with the first cross-section and/or second cross-section.

The distal end of the elongate portion, potentially including the adjacent part of the elongate portion, may provide a flared section. The flared section may be flared in a lateral and/or medial direction. The flared section may be flared in the direction of the first axis relative to the second axis, potentially in the direction away from the first axis too.

The inserter device may have a tool disengaged state. The inserter device may have a tool engaged state. The inserter device may have an instrument non-contacting state. The inserter device may have an instrument contacting state. The inserter device may have an alternative orientation state. The inserter device may have an instrument inserting state.

The tool disengaged state may provide the inserter device spaced from the tool. The tool engaged state may provide the inserter device mounted on the tool. The tool engaged state may provide restraint against movement of the inserter device away from the tool axially.

The inserter device may pass through a first transition stage between the tool disengaged state and the tool engaged state. The inserter device and the tool may be advanced axially toward one another in the first transition stage.

The instrument disengaged state may provide the inserter device mounted on the tool and/or spaced from the instrument.

The instrument contacting state may provide a part of the inserter device, for instance the second mounting location in contact with a part of the instrument. The second mounting location may be partially or fully inserted into the instrument. The instrument contacting state may provide no restraint against movement of the inserter device and/or instrument away from one another axially. The instrument contacting state may provide restraint against rotation of the inserter device relative to the instrument.

The inserter device may pass through a second transition stage between the instrument disengaged state and the instrument contacting state. The inserter device and the instrument may be advanced axially toward one another in the second transition stage. The inserter device and the instrument may not be rotated relative to one another in the second transition stage.

The inserter device may have one or more alternative orientation states, particularly whilst in the instrument contacting state. The one or more alternative orientation states may be a different angle[s] of the longitudinal axis of the inserter device relative to the direction of movement during the second transition stage and/or relative to the longitudinal axis of the instrument and/or relative to an axis or plane parallel to the longitudinal axis of the instrument.

The inserter device may have a range of alternative orientation states in an articulating plane. The articulating plane may extend medially-laterally. The articulating plane may extend between the first axis and the second axis of the curved portion.

The inserter device may have a second limited range of alternative orientation states in another plane, for instance a non-articulating plane. The non-articulating plane may extend anteriorly-posteriorly. The non-articulating plane may extend perpendicular to that plane extending between the first axis and the second axis of the curved portion.

The inserter device may have a series of alternative orientation states, particularly in the articulating plane, over a range of orientation states spanning more than 10° of arc, for instance more than 20° of arc, such as more that 30° of arc or even up to 45° of arc. The inserter device may have a series of alternative orientation states, particularly in the articulating plane, over a range of orientation states spanning less than 60° of arc, for instance less than 50° of arc. The inserter device may have a series of alternative orientation states, particularly in the articulating plane, over a range of 0° to 10° in a medial direction relative to the longitudinal axis of the instrument and/or relative to an axis or plane parallel to the longitudinal axis of the instrument. The inserter device may have a series of alternative orientation states, particularly in the articulating plane, over a range of 20° to 50°, for instance 30° to 40°, in a lateral direction relative to the longitudinal axis of the instrument and/or relative to an axis or plane parallel to the longitudinal axis of the instrument. The one or more alternative orientation states may provide restraint against movement of the inserter device and/or instrument away from one another axially.

The inserter device may have a limited series of second limited alternative orientation states, particularly in the non-articulating plane, over a range of orientation states spanning less than 10° of arc, for instance less than 5° of arc, such as less than 3° of arc or even less than 1° of arc. The inserter device may have a limited series of second limited alternative orientation states, particularly in the non-articulating plane, over a range of less than 10° of arc, for instance less than 5° of arc, such as less than 3° of arc or even less that 1° of arc in a posterior and/or anterior direction.

The inserter device may transition from one alternative orientation state, particularly in the articulating plane, to another alternative orientation state by rotating the inserter device relative to the instrument, for instance in the medial direction or lateral direction.

The instrument inserting state may provide an axial load on the inserter device towards the instrument. The axial load may be applied via the tool. The axial load may be constant or may vary. The instrument inserting state may provide relative movement of the instrument and the patient.

The body, for instance the body portion, may be provided with one or more visible indicators of the orientation of the first mounting location. The one or more visible marks may indicate alignment or misalignment with the tool, for instance rotationally. The visible indicators may be one or more surfaces on the body portion. The visible indicators may be outside of the instrument and/or visible in all states and stages.

The first aspect may include any of the features, possibilities or options set out elsewhere in this document, including in the other aspects.

According to a second aspect of the invention there is provided a kit comprising:
a surgical instrument inserter device according to the first aspect and
a tool for attachment to the first mounting location of the surgical instrument inserter device.

The tool may further include a handle, for instance towards the proximal end of the tool. The tool may further include one or more abutment surfaces, for instance one or more radially extending elements or flanges, particularly at the proximal end of the tool. The one or more abutment surfaces may be adapted for impact by a further tool.

The tool may be provided with an inserter device mounting location. The inserter device mounting location may be a complimentary mounting location to the first mounting location of the inserter device. The inserter device mounting location may include a bore. The bore may extend into the tool. The inserter device mounting location may include a releasable retention element, such as a latch mechanism. The inserter device mounting location may be adapted to connect, for instance receive, a proximal end of the inserter device.

The inserter device mounting location may have an inserter device engaged state and an inserter device disengaged state. The inserter device mounting location may transition from the inserter device engaged state to the inserter device disengaged state and/or from the inserter device disengaged state to the inserter device engaged state by axial movement of the inserter device and tool relative to one another. The inserter device mounting location may transition from the inserter device engaged state to the inserter device disengaged state and/or from the inserter device disengaged state to the inserter device engaged state by operation of a releasable engagement.

The tool may be adapted to perform one or more further roles, besides contact with the inserter device. The tool could also serve as an instrument removal device for use in extracting stems.

The second aspect of the invention may include any of the features, possibilities or options set out elsewhere in this document in relation to the inserter device, including in the first aspect in particular. The second aspect may include any of the features, possibilities or options set out elsewhere in this document, including in the other aspects.

According to a third aspect of the disclosure, which is provided for illustrative purposes only and does not form part of the present invention, there is provided a surgical instrument, the surgical instrument including a tapered body extending distally from a first end to a second end, one or more sections of the tapered body being provided with a plurality of cutting teeth, a superior surface of the tapered body being provided with an instrument mounting location, wherein the mounting location is provided within an opening and wherein the opening includes one or more contact surfaces, at least one of the contact surfaces being a non-planar contact surface.

The instrument may be an implant.

The instrument may have a superior portion. The superior portion may be provided with a series of teeth and/or recesses, particularly teeth alternating with recesses. The instrument may have a calcar face. The calcar face may be provided with a projection, such as a spigot, for instance for receiving a trial or a part thereof. The calcar surface may be provided with the opening.

The opening may be a slot. The slot may be a closed end slot. The slot may be an open-end slot, for instance at the medial end. The opening may be an aperture. The opening may be an aperture which is connected to a second aperture in an inferior face of the broach. The opening may be partially defined by one or more perimeter wall[s]. The opening may be partially defined by one or more base walls.

The opening may provide a recess into the instrument, for instance inferior to the perimeter wall[s] defining the opening. The chamber may be partially defined by one or more side walls and/or a base wall.

The opening may have a width, considered anteriorly-posteriorly, which is less than the length of the opening, considered medially-laterally. The opening may have a second extent, considered anteriorly-posteriorly, which is less than the first extent, considered medially-laterally.

The first extent of the opening may be greater than the first extent of the inserter device. The second extent of the opening may be greater than the second extent of the inserter device.

The opening may provide one or more contact surfaces for one or more surfaces of the second mounting location of the inserter device. The one or more contact surfaces may provide articulation surfaces, for instance to allow rotation of the inserter device relative to the instrument. The opening may provide one or more non-planar surface sections, for instance complimentary to one or more non-planar surface sections of the inserter device. The base of the opening may provide one or more non-planar surface sections, for instance complimentary to one or more non-planar surface sections of the inserter device. The end, for instance the lateral end of the opening may provide one or more non-planar surface sections, for instance complimentary to one or more non-planar surface sections of the inserter device. The side walls of the opening may provide one or more non-planar surface sections, for instance complimentary to one or more non-planar surface sections of the inserter device.

The opening may limit the range of variations possible in the axial alignment of the tool and/or inserter device, for instance by abutment.

The instrument may be provided with one or more teeth and/or recesses, for instance on the anterior and posterior faces. The instrument may be provided with tooth and recess free space on or near the lateral shoulder face, for instance provided with alpha/numerical markings

The third aspect may include any of the features, possibilities or options set out elsewhere in this document, including in the other aspects.

According to a fourth aspect of the disclosure, which is provided for illustrative purposes only and does not form part of the present invention, there is provided a method inserting a surgical instrument from a patient, the method comprising:
1) providing a surgical instrument inserter device, the inserter device comprising:
   a body;
   a first mounting location provided at the proximal end of the body;
   a second mounting location provided at the distal end of the body;
   wherein the second mounting location includes a contact element for the surgical instrument to be inserted in use, the contact element having one or more non-planar contact surface sections.
2) inserting the contact element of the inserter device into an opening in the surgical instrument to provide an instrument contacting state;
3) decreasing the separation between the inserter device and the patient to insert the surgical instrument in the patient.

The method may include a tool disengaged state for the inserter device. The method may include a tool engaged state.

The method may include an instrument disengaged state. The method may include an instrument contacting state.

The method may include an alternative orientation state. The method may include an instrument inserting state.

The tool disengaged state may provide the inserter device spaced from the tool.

The tool engaged state may provide the inserter device mounted on the tool. The tool engaged state may provide restraint against movement of the inserter device away from the tool axially. The method may further include, connecting a tool to the inserter device to provide the tool engaged state. The tool may be connected to the inserter device before step 2) is commenced.

The method may include the inserter device passing through a first transition stage between the tool disengaged state and the tool engaged state. The method may include the inserter device and the tool advanced axially toward one another during the first transition stage.

The instrument disengaged state may provide the inserter device mounted on the tool and/or spaced from the instrument.

The instrument contacting state may provide a part of the inserter device, for instance the second mounting location in contact with a part of the instrument. The second mounting location may be partially or fully inserted into the instrument. The instrument contacting state may provide no restraint against movement of the inserter device and/or instrument away from one another axially. The instrument contacting state may provide restraint against rotation of the inserter device relative to the instrument.

The method may include the inserter device passing through a second transition stage between the instrument disengaged state and the instrument contacting state. The method may include the inserter device and/or the instrument being advanced axially toward one another during the second transition stage. The inserter device and the instrument may not be rotated relative to one another during the second transition stage.

The method may include the inserter device having one or more alternative orientation states, particularly whilst in the instrument contacting state. The one or more alternative orientation states may be a different angle[s] of the longitudinal axis of the inserter device relative to the direction of movement during the second transition stage and/or relative to the longitudinal axis of the instrument and/or relative to an axis or plane parallel to the longitudinal axis of the instrument.

The method may include the inserter device having a range of alternative orientation states in an articulating plane. The articulating plane may extend medially-laterally. The articulating plane may extend between the first axis and the second axis of the curved portion.

The method may include the inserter device having a second limited range of alternative orientation states in another plane, for instance a non-articulating plane. The non-articulating plane may extend anteriorly-posteriorly. The non-articulating plane may extend perpendicular to that plane extending between the first axis and the second axis of the curved portion.

The method may include the inserter device having a series of alternative orientation states over a range of orientation states spanning more than 10° of arc, for instance more than 20° of arc, such as more that 30° of arc or even up to 45° of arc. The method may include the inserter device having a series of alternative orientation states over a range of orientation states spanning less than 60° of arc, for instance less than 50° of arc. The method may include the inserter device having a series of alternative orientation states over a range of 0° to 10° in a medial direction relative to the longitudinal axis of the instrument and/or relative to an axis or plane parallel to the longitudinal axis of the instrument. The method may include the inserter device having a series of alternative orientation states over a range of 20° to 50°, for instance 30° to 40°, in a lateral direction relative to the longitudinal axis of the instrument and/or relative to an axis or plane parallel to the longitudinal axis of the instrument. The method may include the one or more alternative orientation states providing restraint against movement of the inserter device and/or instrument away from one another axially.

The method may include the inserter device having a limited series of second limited alternative orientation states, particularly in the non-articulating plane, over a range of orientation states spanning less than 10° of arc, for instance less than 5° of arc, such as less than 3° of arc or even less that 1° of arc. The method may include the inserter device having a limited series of second limited alternative orientation states, particularly in the non-articulating plane, over a range of less than 10° of arc, for instance less than 5° of arc, such as less than 3° of arc or even less that 1° of arc in a posterior and/or anterior direction.

The method may include the inserter device transitioning from one alternative orientation state, particularly in the articulation plane, to another alternative orientation state by rotating the inserter device relative to the instrument, for instance in the medial direction or lateral direction.

The method may include the instrument inserting state in a plurality of different alternative orientation states.

The method may include the instrument inserting state providing an axial load on the inserter device towards the instrument. The axial load may be applied via the tool. The axial load may be constant or may vary. The instrument inserting state may provide relative movement of the instrument and the patient.

The fourth aspect may include any of the features, possibilities or options set out elsewhere in this document, including in the other aspects.

### BRIEF DESCRIPTION OF THE DRAWINGS

Various embodiments of the disclosure will now be described, by way of example only, and with reference to the accompanying figures, in which:
Figure 1a is a perspective view of a first embodiment of an inserter device;
Figure 1b is a detail view of the distal end of the inserter device of Figure 2a;
Figure 2a is a perspective view of a second embodiment of an inserter device connected to a tool and illustrating the range of articulation;
Figure 2b is a perspective view of the inserter device of Figure 2a, provided transmitting torque to an implant.

### DETAILED DESCRIPTION OF THE DRAWINGS

While the concepts of the present disclosure are susceptible to various modifications and alternative forms, specific embodiments thereof have been shown by way of example in the drawings and will be described herein in detail. It should be understood, however, that there is no intent to limit the concepts of the present disclosure to the particular forms disclosed, but on the contrary, the intention is to cover all modifications and alternatives consistent with the present disclosure and the appended claims.

Terms representing anatomical references, such as anterior, posterior, medial, lateral, superior, inferior, et cetera, may be used throughout the specification in reference to the orthopaedic implants or prostheses and surgical instruments described herein as well as in reference to the patient's natural anatomy. Such terms have well-understood meanings in both the study of anatomy and the field of orthopaedics. Use of such anatomical reference terms in the written description and claims is intended to be consistent with their well-understood meanings unless noted otherwise.

In hip arthroplasty, a femoral component of the prosthesis must be successfully introduced into the prepared proximal end of the femur. Press-fit implantation is frequently used, but other forms of implantation can be deployed. Inserter devices, such as impactors, can be used for this purpose.

Numerous designs provide for a linear shaft for the device which engages with the implant so that the longitudinal axis of the shaft and of the implant are aligned. This is beneficial for effective energy transfer from the shaft to the implant and/or to reduce the risk of damage to the implant and/or shaft.

In some instances, linear shafts encounter difficulties with their use due to the patient's anatomy and/or the degree of exposure provided at the surgical site limiting or preventing axial alignment.

The present disclosure seeks to provide an ability to be able to accommodate a wider range of patient anatomies and degrees of site exposure. The present disclosure seeks to provide effective force transmission between the shaft and implant across a wide range of orientations. The present disclosure seeks to provide a secure contact between the shaft and the implant.

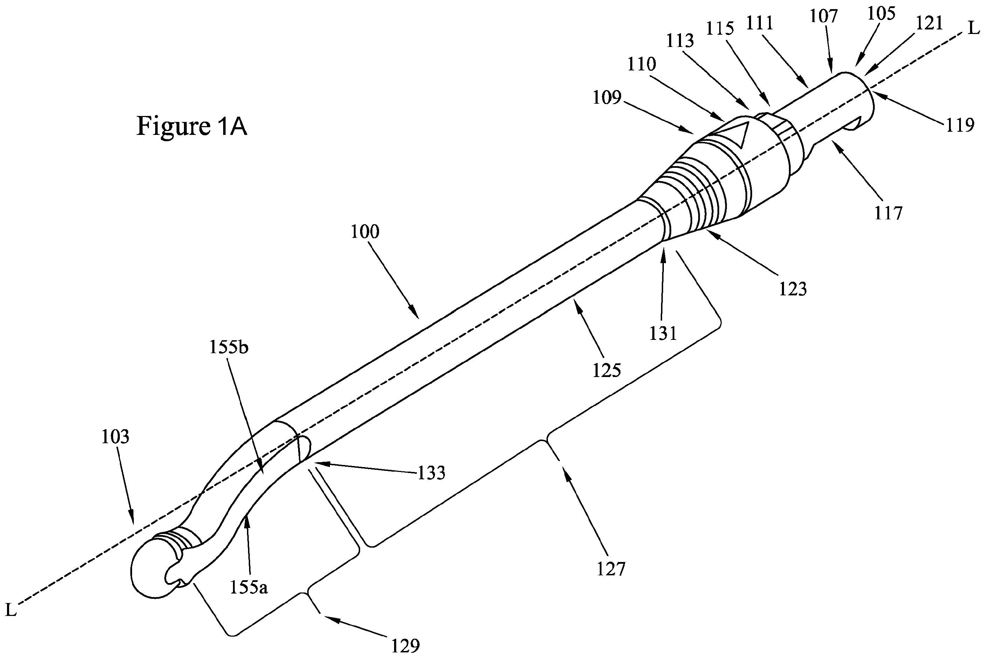

A first embodiment of an inserter device 100 according to the invention **is** illustrated in Figure 1a. The inserter device 100 is provided with a distal end 103 and a proximal end 105.

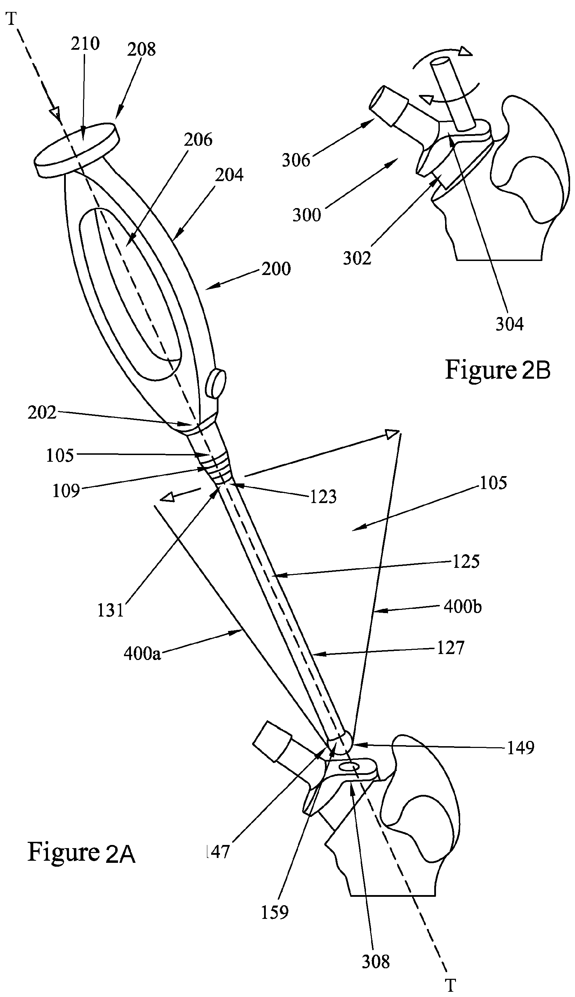

As seen in Figure 2b, the proximal end 105 provides a mounting location 107 for connecting the inserter device 100 to the distal end 202 of a tool 200, for instance an impacter device. Various configurations for the mounting location 107 are possible, but each have a suitable configuration for engagement with the configuration of the distal end 202 of the tool 200.

In the illustrated configuration of Figure 1a, the proximal end 105 is provided with a body portion 109 which mounts a reduced diameter projecting element 111. The base 113 of the projecting element 111 has a flat surface 115 which assists with the correct orientation of the inserter device 100 when mounted on the tool 200. The flat surface 115 is received in opposition to a similar profile flat within a bore in the distal end 202 of the tool 200. The projecting element 111 is generally cylindrical, but is provided with a cut out 117. A cylindrical cap 119 forms the proximal end 121 of the projecting element 111. The cap 119 cooperates with a latch type mechanism of the tool 200, which latch type mechanism enters the cut out 117. This interaction restrains axial movement of the tool 200 and the inserter device 100 relative to one another.

Indicia 110 are provided on the body portion 109 to show the alignment to seek of the inserter device 100 with the tool 200.

A transition section 123 provides a taper from the body portion 109 and its profile down to a reduced profile of a shaft 125.

The shaft 125 is provided with a linear section 127 and a curved section 129. The linear section 127 is provided closer to the proximal end 105 of the inserter device 100 than the curved section 125. The curved section 129 is therefore provided closer to the distal end 103 of the inserter device 100 than the linear section 127.

The linear section 127 has a longitudinal axis L-L which is coaxial with the axis of the body portion 109 and projecting element 111. As can be seen in Figure 2b, the longitudinal axis T-T of the tool 200 is also coaxial with this axis L-L.

The cross-section of the linear section 127 is consistent between its junction 131 with the transition section 123 until adjacent the junction 133 between the linear section 127 and the curved section 129. The cross-section is circular through this consistent section. Adjacent the junction 133 through to the junction 133, the cross-section is partially circular, but with two flat faces 155a, 155b [described further below].

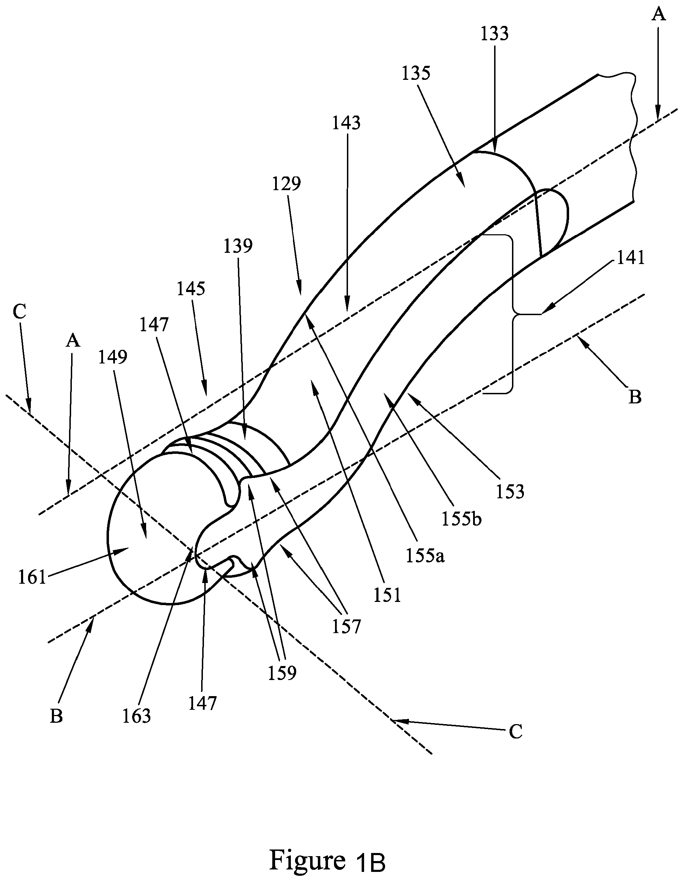

Referring to Fig 1b, the curved section 129 includes a first part 135 having an axis A-A which is coaxial with the longitudinal axis L-L of the shaft 125. The curved section 129 includes a second part 139 having an axis B-B which is parallel to the axis A-A and is also parallel to the axis L-L. The amount of offset 141 between the axis A-A and the axis B-B defines the curve of the curved section 129 and part of the ability to accommodate patient anatomy and/or surgical site exposure variations. The curve part 143, intermediate the first part 135 and the second part 139, first curves away from the axis A-A and then curves back to the axis B-B.

The distal end 145 of the curved section 129 provides a junction 147 between the curved section 129 and an end element 149.

The cross-section of the curved section 129 is consistent between the junction 133 with the linear section 127 until adjacent the junction 147. The cross-section is partially circular, with part-circular medial section 151, part-circular lateral section 153 and two flat face sections 155a, 155b, one on each side of the curved section 129. The two flat sections 155a, 155b extend proximally slightly past the junction 133.

As best seen in Figure 1b, the flat surfaces 155a, 155b are present through to the junction 147. From a cross-section location 157 adjacent the junction 147 through to the junction 147, however, the part-circular section 153 and the part-circular section 155 cease to be part-circular and instead gradually increase to provide a flared section 159 having an elongated [anterior-posterior] cross-section. The role of the flared section 159 is described below.

As seen in both Figures 1a and 1b, the inserter device 100 is provided at its distal end 103 with an end element 149.

In the embodiment shown, the end element 149 is in the shape of part of a compressed sphere. The direction of compression is along axis C-C. The end element 149 has an equator 161 extending around the end element 149 in a plane intersecting axis A-A and also intersecting axis B-B. The end element 149 may have polar regions 163 about axis C-C and/or about an axis perpendicular to the equator 161. The polar regions 163 may be flattened as show in Figure 1a and 1b. The curved sections of the end element 149 allow for the inserter device 100 to articulate relative to the implant, as will be described in further detail below. The compressed or flattened sections of the end element 149 provide for a more constrained orientation of the inserter device 100 relative to the implant in that direction. The portion of the flat face sections 155a, 155b which are within the implant in use, which may include the compressed or flattened sections of the end element 149, allow for the application of rotational torque to the implant to check the stability of the implant in the patient.

A variety of shapes for the end element are possible, whilst providing curved surfaces offering the desired articulation in the desired directions and/or whilst offering less curved or flatter surfaces offering greater constraint on articulation in other desired directions. For instance, in an embodiment, relative to a center-point of the end element 149, the external surface of the end element 149 is defined by a first radius in a plane intersection axis A-A and also intersecting axis B-B. This may be a lateral-medial extending plane. The external surface is define by a second radius in a plane intersection axis B-B and extending perpendicular to axis A-A. This may be an anterior-posterior plane. The second radius is less than the first radius. Between the two planes, the radii may transition from the second radius up to the first radius to give intermediate curvature.

As seen in Figure 2a, the inserter device 100 in use, has its proximal end 105 connected to the distal end 202 of a tool 200. The tool 200 includes a handle section 204 with a through aperture 206 to allow the surgeon to hold the tool 200 from a variety of directions. The proximal end 208 of the tool 200 is provided with a surface 210 to which impacts or pressure can be applied to advance the implant [not shown] into the patient using the inserter device 100. The tool 200 has an axis T-T.

The tool 200 can be used with a range of interchangeable inserter devices 100, such as the illustrated in Figures 1a and 1b. In Figure 2a a second embodiment of the inserter device 100 is illustrated. This has an end element 149 with the shape, configuration and operation described above. However, in this case whilst the articulation is provided, the inserter device 100 is a purely linear one. Thus, the inserter device 100 has a body portion 109 [and associated elements described in detail above] and a transition section 123 down to a reduced profile of a shaft 125. The shaft 125 is provided with a linear section 127 only in this embodiment. The linear section 127 has a longitudinal axis L-L which is coaxial with the axis of the body portion 109 and projecting element 111 and with the axis T-T of the tool 200.

The cross-section of the linear section 127 is consistent between its junction 131 with the transition section 123 until closer to the junction 147 between the linear section 127 and end element 149. The cross-section is circular through this consistent section, but closer to the junction 147, the cross-section is partially circular, but with two flat faces 155a, 155b again. Adjacent the junction 147, a flared section 159 having an elongated [anterior-posterior] cross-section is once again provided.

In the second embodiment too, the end element 149 is in the shape of part of a compressed sphere, as before. The direction of compression is along axis C-C as before and the other end element 149 features are also present.

In Figure 2a, the end element 149 is shown advanced axially towards the calcar face 302 of an implant 300.

The implant has a superior portion 304 visible in Figure 2a and 2b, which along with the rest of the implant [not shown] is provided with a series of grooves alternating with recesses to assist in the retention of the implant. The calcar face 302 of the implant 300 extends medially-laterally, when inserted in the femur. The calcar face 302 is provided with a projection 306 which receives a further implant [not shown].

The calcar surface 302 is provided with a slot 308 which extends inferiorly into the implant 300. The slot 308 is defined by perimeter wall[s] and by a base wall . The slot 308 has a width which is a clearance size greater that the width of the end element 149 along axis C-C. The slot 308 has a width, considered anteriorly-posteriorly A-P, which is less than the length of the slot 308, considered medially-laterally M-L.

As shown in Figure 2b, the end element 149 is inserted into the slot 308 with the flats 155a, 155b and the flattened polar regions on the end element 149 aligned anteriorly-posteriorly A-P and so the reduced "width" of the end element 149 in that orientation, due to the flats 155a, 155b, and the flattened polar regions, allows the end element 149 to enter the slot 308 and then contact one or more parts of the walls of the slot 308.

If the flats 155a, 155b on the end element 149 are presented facing laterallymedially L-M, then the full "width" of the end element 149 in that orientation, due to the fuller part of the compressed sphere of the end element 149 being presented, means that the end element 149 cannot enter the slot 308.

As shown in Figure 2b, the end element 149 is within the slot 308 and the flared section 159 is outside of the slot 308.

In the inserted state of Figure 2b, any attempt to move the tool 200 and the inserter device 100 in a proximal axial direction will result in the transition back from the inserted state to the disengaged state as there is nothing to resist that movement and separation.

The slot 308 has an internal profile, defined by the perimeter walls and base wall, that provides an at least partially complementary surface or surfaces that contact the end element 149 of the inserter device 100. As described above, the shape and configuration provided for the end element 149 is a compressed sphere and so a similar female surface in the slot 308 allows a range of alternative orientation states for the inserter device 100 and tool 200 relative to the implant 300. Referring to Figure 2a, the range is illustrated by the grey shaded arc 400a to 400b. The range extends medially and laterally over some 40° of arc. Little to no articulation is allowed in other directions, such as anteriorly-posteriorly, due, for instance, to the flattened polar regions of the end element 149 and the corresponding parts of the slot walls forming more planar surfaces which do not support relative articulation.

The articulation facilitated allows the tool 200 and/or inserter device 100 to avoid patient anatomy and/or work within the limitations of the surgical site exposure.

Referring to the inserted state of Figure 2b, if impacts are applied to the surface 210 of the tool 200 then that force will be communicated through the tool 200 to the inserter device 100 and hence to the implant 300. The profile of the slot 308 and the end element 149 are designed to be complimentary to one another to a large extent and so the forces are conveyed over a large area. This reduces the chances of damage to the inserter device 100 and/or the implant 300. The profile of the slot 308 and the end element 149 are designed to be complimentary to one another to a large extent over the full range of alternative orientation states and so optimized force transfer is provided even if the axis of the tool 200 and/or inserter device 100 is not aligned with the axis of the implant 300.

Referring to the inserted state of Figure 2b, if the tool 200 and the inserter device 100 are rotated, in either direction, then rotational torque is applied to the implant as the width of the slot 308 is so close to the width of the end element 149 so as not to allow material relative rotation of the inserter device 100 and the implant 300.

As the inserter device 100 and hence the end element 149 reaches the limit of the arc of alternative orientations, then the flared section 159 will abut the perimeter of the slot 308 and hence provide tactile feedback to the surgeon that the full extent of inclination of the inserter device relative to the implant has been reached.

While the disclosure has been illustrated and described in detail in the drawings and foregoing description, such an illustration and description is to be considered as exemplary and not restrictive in character, it being understood that only illustrative embodiments have been shown and described and that all changes and modifications that come within the scope of the invention as defined by the claims are desired to be protected.

There are a plurality of advantages of the present disclosure arising from the various features of the apparatus, system, and method described herein. It will be noted that alternative embodiments of the apparatus, system, and method of the present disclosure may not include all of the features described yet still benefit from at least some of the advantages of such features. Those of ordinary skill in the art may readily devise their own implementations of the apparatus and system that incorporate one or more of the features of the present invention and fall within the scope of the invention as defined by the claims.

## Claims

1. A surgical instrument inserter device (100), the inserter device (100) comprising:
a body;
a first mounting location (107) provided at the proximal end (105) of the body;
a second mounting location provided at the distal end (103) of the body;
wherein the second mounting location includes a contact element (149) for the surgical instrument to be inserted in use, the contact element (149) having one or more non-planar contact surface sections;
**characterised in that** one or more or all of the contact surface sections are provided by a deformed partial sphere having reduced radii in a first set of directions compared with radii in a second set of directions.

2. The inserter device (100) of claim 1, wherein the contact element (149) provides a single non-planar contact surface.

3. The inserter device (100) of claim 1 or claim 2, wherein the non-planar contact surface[s] are curved contact surface[s].

4. The inserter device (100) of any preceding claim wherein one or more or all of the non-planar contact surface sections are articulation surfaces.

5. The inserter device (100) of any preceding claim, wherein one or more or all of the contact surface sections are provided by a compressed sphere, with the compression along one axis (C).

6. The inserter device (100) of any preceding claim, wherein the contact element (149) includes one or more other contact surface sections (155a, 155b), the one or more other contact surface sections (155a 155b) being one or more near planar or planar other contact surface sections (155a, 155b).

7. The inserter device (100) of claim 6, wherein the one or more other contact surface sections (155a, 155b) are sections of the same continuous surface as the one or more non-planar contact surface sections are part of.

8. The inserter device (100) of any preceding claim, wherein the contact element (149) comprises two other contact surface sections (155a, 155b) that are flattened compared with the curvature of the one or more non-planar contact surface sections.

9. The inserter device (100) of claim 8, wherein the two flattened other contact surface sections (155a, 155b) are in opposition to one another and are on posterior and anterior sides of the inserter device (100).

10. The inserter device (100) of any preceding claim, where the contact element (149) has a first extent and a second extent, wherein the first extent is between a first pair of points on opposite sides of the contact element (149) and wherein the second extent is between a second different pair of points on different opposite sides of the contact element (149), wherein the first extent is greater than the second extent.

11. The inserter device (100) of any preceding claim, wherein the body includes a body portion (109) and includes an elongate portion (127), together with a transition portion (123) that connects the body portion (109) to the elongate portion (127).

12. The inserter device (100) of any preceding claim, wherein the first mounting location (107) is adapted to connect the inserter device (100) to the distal end (202) of a tool (200).

13. A kit comprising:
a surgical instrument inserter device (100) according to claim 1 and a tool (200) for attachment to the first mounting location (107) of the surgical instrument inserter device (100).

14. The kit of claim 13, wherein the tool (200) further includes a handle (204) and/or one or more abutment surfaces (210) and/or is adapted for impact by a further tool.

15. The kit of claim 13 or claim 14, wherein the kit further includes a plurality of different profile inserter devices (100).

## Patentansprüche

1. Eine Einsetzvorrichtung (100) für ein chirurgisches Instrument, wobei die Einsetzvorrichtung (100) Folgendes beinhaltet:
einen Körper;
eine erste Montagestelle (107), die an dem proximalen Ende (105) des Körpers bereitgestellt ist;
eine zweite Montagestelle, die an dem distalen Ende (103) des Körpers bereitgestellt ist;
wobei die zweite Montagestelle ein Kontaktelement (149) für das im Gebrauch einzusetzende chirurgische Instrument umfasst, wobei das Kontaktelement (149) einen oder mehrere nicht planare Kontaktflächenteilabschnitte aufweist;
**dadurch gekennzeichnet, dass** einer oder mehrere oder alle der Kontaktflächenteilabschnitte durch eine verformte Teilkugel bereitgestellt werden, die reduzierte Radien in einem ersten Satz von Richtungen im Vergleich zu Radien in einem zweiten Satz von Richtungen aufweist.

2. Einsetzvorrichtung (100) gemäß Anspruch 1, wobei das Kontaktelement (149) eine einzelne nicht planare Kontaktfläche bereitstellt.

3. Einsetzvorrichtung (100) gemäß Anspruch 1 oder Anspruch 2, wobei die nicht planare[n] Kontaktfläche[n] gekrümmte Kontaktfläche[n] ist/sind.

4. Einsetzvorrichtung (100) gemäß einem der vorhergehenden Ansprüche, wobei einer oder mehrere oder alle der nicht planaren Kontaktflächenteilabschnitte Artikulationsflächen sind.

5. Einsetzvorrichtung (100) gemäß einem der vorhergehenden Ansprüche, wobei einer oder mehrere oder alle der nicht planaren Kontaktflächenteilabschnitte durch eine komprimierte Kugel bereitgestellt werden, mit der Kompression entlang einer Achse (C).

6. Einsetzvorrichtung (100) gemäß einem der vorhergehenden Ansprüche, wobei das Kontaktelement (149) einen oder mehrere andere Kontaktflächenteilabschnitte (155a, 155b) umfasst, wobei der eine oder die mehreren anderen Kontaktflächenteilabschnitte (155a, 155b) ein oder mehrere nahezu planare oder planare andere Kontaktflächenteilabschnitte (155a, 155b) sind.

7. Einsetzvorrichtung (100) gemäß Anspruch 6, wobei der eine oder die mehreren anderen Kontaktflächenteilabschnitte (155a, 155b) Teilabschnitte derselben durchgehenden Fläche sind, zu der der eine oder die mehreren nicht planaren Kontaktflächenteilabschnitte gehören.

8. Einsetzvorrichtung (100) gemäß einem der vorhergehenden Ansprüche, wobei das Kontaktelement (149) zwei andere Kontaktflächenteilabschnitte (155a, 155b) beinhaltet, die im Vergleich zu der Krümmung des einen oder der mehreren nicht planaren Kontaktflächenteilabschnitte abgeflacht sind.

9. Einsetzvorrichtung (100) gemäß Anspruch 8, wobei die zwei abgeflachten anderen Kontaktflächenteilabschnitte (155a, 155b) einander gegenüberliegen und sich auf posterioren und anterioren Seiten der Einsetzvorrichtung (100) befinden.

10. Einsetzvorrichtung (100) gemäß einem der vorhergehenden Ansprüche, wobei das Kontaktelement (149) eine erste Erstreckung und eine zweite Erstreckung aufweist, wobei die erste Erstreckung zwischen einem ersten Paar von Punkten auf gegenüberliegenden Seiten des Kontaktelements (149) liegt und wobei die zweite Erstreckung zwischen einem zweiten unterschiedlichen Paar von Punkten auf unterschiedlichen gegenüberliegenden Seiten des Kontaktelements (149) liegt, wobei die erste Erstreckung größer als die zweite Erstreckung ist.

11. Einsetzvorrichtung (100) gemäß einem der vorhergehenden Ansprüche, wobei der Körper einen Körperabschnitt (109) umfasst und einen länglichen Abschnitt (127) umfasst, zusammen mit einem Übergangsabschnitt (123), der den Körperabschnitt (109) mit dem länglichen Abschnitt (127) verbindet.

12. Einsetzvorrichtung (100) gemäß einem der vorhergehenden Ansprüche, wobei die erste Montagestelle (107) angepasst ist, um die Einsetzvorrichtung (100) mit dem distalen Ende (202) eines Werkzeugs (200) zu verbinden.

13. Ein Kit, das Folgendes beinhaltet:
eine Einsetzvorrichtung (100) gemäß Anspruch 1 für ein chirurgisches Instrument und ein Werkzeug (200) zur Befestigung an der ersten Montagestelle (107) der Einsetzvorrichtung (100) für ein chirurgisches Instrument.

14. Kit gemäß Anspruch 13, wobei das Werkzeug (200) ferner einen Griff (204) und/oder eine oder mehrere Anlageflächen (210) umfasst und/oder für einen Schlag durch ein weiteres Werkzeug angepasst ist.

15. Kit gemäß Anspruch 13 oder Anspruch 14, wobei das Kit ferner eine Vielzahl von Einsetzvorrichtungen (100) mit unterschiedlichen Profilen umfasst.

## Revendications

1. Un dispositif inserteur (100) pour instrument chirurgical, le dispositif inserteur (100) comprenant :
un corps ;
un premier emplacement de montage (107) fourni à l'extrémité proximale (105) du corps ;
un deuxième emplacement de montage fourni à l'extrémité distale (103) du corps ;
le deuxième emplacement de montage incluant un élément de contact (149) pour l'instrument chirurgical à insérer en cours d'utilisation, l'élément de contact (149) présentant une ou plusieurs sections de surface de contact non planes ;
**caractérisé en ce qu'**une ou plusieurs ou toutes les sections de surface de contact sont fournies par une sphère partielle déformée présentant des rayons réduits dans un premier ensemble de directions par comparaison à des rayons dans un deuxième ensemble de directions.

2. Le dispositif inserteur (100) de la revendication 1, dans lequel l'élément de contact (149) fournit une surface de contact non plane unique.

3. Le dispositif inserteur (100) de la revendication 1 ou de la revendication 2, dans lequel la ou les surfaces de contact non planes sont une ou des surfaces de contact courbées.

4. Le dispositif inserteur (100) de n'importe quelle revendication précédente dans lequel une ou plusieurs ou toutes les sections de surface de contact non planes sont des surfaces d'articulation.

5. Le dispositif inserteur (100) de n'importe quelle revendication précédente, dans lequel une ou plusieurs ou toutes les sections de surface de contact sont fournies par une sphère comprimée, avec la compression le long d'un axe (C).

6. Le dispositif inserteur (100) de n'importe quelle revendication précédente, dans lequel l'élément de contact (149) inclut une ou plusieurs autres sections de surface de contact (155a, 155b), les une ou plusieurs autres sections de surface de contact (155a, 155b) étant une ou plusieurs autres sections de surface de contact quasi planes ou planes (155a, 155b).

7. Le dispositif inserteur (100) de la revendication 6, dans lequel les une ou plusieurs autres sections de surface de contact (155a, 155b) sont des sections de la même surface continue que celle dont font partie les une ou plusieurs sections de surface de contact non planes.

8. Le dispositif inserteur (100) de n'importe quelle revendication précédente, dans lequel l'élément de contact (149) comprend deux autres sections de surface de contact (155a, 155b) qui sont aplaties par comparaison à la courbure des une ou plusieurs sections de surface de contact non planes.

9. Le dispositif inserteur (100) de la revendication 8, dans lequel les deux autres sections de surface de contact aplaties (155a, 155b) sont en opposition l'une à l'autre et sont sur des côtés postérieur et antérieur du dispositif inserteur (100).

10. Le dispositif inserteur (100) de n'importe quelle revendication précédente, où l'élément de contact (149) présente une première étendue et une deuxième étendue, dans lequel la première étendue est entre une première paire de points sur des côtés opposés de l'élément de contact (149) et dans lequel la deuxième étendue est entre une deuxième paire différente de points sur des côtés opposés différents de l'élément de contact (149), dans lequel la première étendue est supérieure à la deuxième étendue.

11. Le dispositif inserteur (100) de n'importe quelle revendication précédente, dans lequel le corps inclut une portion de corps (109) et inclut une portion allongée (127), conjointement avec une portion de transition (123) qui relie la portion de corps (109) à la portion allongée (127).

12. Le dispositif inserteur (100) de n'importe quelle revendication précédente, dans lequel le premier emplacement de montage (107) est conçu pour relier le dispositif inserteur (100) à l'extrémité distale (202) d'un outil (200).

13. Un kit comprenant :
un dispositif inserteur (100) pour instrument chirurgical selon la revendication 1 et un outil (200) pour fixation au premier emplacement de montage (107) du dispositif inserteur (100) pour instrument chirurgical.

14. Le kit de la revendication 13, dans lequel l'outil (200) inclut en outre une poignée (204) et/ou une ou plusieurs surfaces de butée (210) et/ou est conçu pour un impact par un outil supplémentaire.

15. Le kit de la revendication 13 ou de la revendication 14, le kit incluant en outre une pluralité de dispositifs inserteurs (100) de profils différents.
